# EUROPEAN PATENT APPLICATION

(11) **EP 1 541 055 A2**
(43) Date of publication of application: **15.06.2005**
(21) Application number: 04425902.6
(22) Date of filing: 30.11.2004
(51) Int. Cl.: A45D 40/28

(54) **Universal applicator mono-use for pastes, salves, creams, powders, liquids and other susceptible substances of manual application**

(30) Priority: 02.12.2003 IT CE20030011 U
(71) Applicant: Romeo, Roberto, 81100 Caserta (IT); Silberstein, Beniamino, 81100 Caserta (IT); Milani, Enrico, 81100 Caserta (IT)
(72) Inventor: Romeo, Roberto, 81100 Caserta (IT); Silberstein, Beniamino, 81100 Caserta (IT); Milani, Enrico, 81100 Caserta (IT)
(74) Representative: Gervasi, Gemma, Dr.

(57) **Abstract**

The invention consists in a disposable item, made in natural or synthetic material suitable to be manufactured and produced in shape of film, with proper size and shape of frustum of cone with rounded apex, suitable to be put on the forefinger, with an extension useful to be tightened in the hand so that the item will remain anchored to the finger while used. Corresponding to the phalangette, or more, there is a spongy material, with variable density depending from the use to which the item is destined, for the delivery of the substance to be applied, so that it would be unnecessary the resort to traditional tools, without limitation of place, situation, encumbrance and carrying on, avoiding also the direct contact with fingers and non desired pollutions on the receiving area. The item is totally innovative and allows particular effectiveness and comfort in applying to liquid, semi-liquid, doughy, fluid with variable density substances such as pastes, salves, unguents; gels, crèmes, powders, disinfectants, liquids and any other substance susceptible of manual application for massage, rubbing, spreading, tamponing and similar.

The innovative peculiarity of efficaciousness and comfort of application or use stands out whether for functionality, or for using form, or for utilization opportunity. The item is highly reliable under hygienic profile, because it is disposable in sealed wrapping. The wrapping can include, laterally, one or two containers being part of the wrapping itself, where pastes, salves, unguents, gels, crèmes, powders may be included, and/or water, like in the item that could be used for teeth cleaning ( water in one side and tooth paste in the other), or medicinal substances (alcohol in one side and unguents or similar in the other side, in case of medical use to clean wounds or for treating lesions, abrasions, maculas etc..) or other substances and or combinations of substances for different uses. The container. will be flattened to optimise the thickness of the wrapping with a maximum length like that of the wrapping itself.

The substance to be applied could be put, while manufacturing, on or under the head of the item made of spongy material impregnating it or spreading trough the open cells of the sponge. The consistence of the head ( spongy material with open or closed cells, depending from the use to be done) can be in synthetic or natural material, biodegradable or not, hydrosoluble or not. The head could be impregnated and compressed, and expand itself, while using, coming in contact with a liquid. Width and shape of the item could change so to allow to put inside it only one finger or two, like in the case of it being used to clean shoes. The head may be applied to its stand (film) by sticking, soldering, or it may be done by expansion of the same material of which the stand is done. The wrapping may also contain accessories depending from the use the item is done for.

## Description

The invention consists in a disposable item, made in natural or synthetic material suitable to be manufactured and produced in shape of film, with proper size and shape of frustum of cone with rounded apex, suitable to be put on the forefinger, with an extension useful to be tightened in the hand so that the item will remain anchored to the finger while used. Corresponding to the phalangette, or more, there is a spongy material, with variable density depending from the use to which the item is destined, for the delivery of the substance to be applied, so that it would be unnecessary the resort to traditional tools, without limitation of place, situation, encumbrance and carrying on, avoiding also the direct contact with fingers and non desired pollutions on the receiving area. The item has a very small size, so that a wrapping containing one or two items could have approx the same thickness and dimension of a credit card container. The examination of the invention can be done with the help of the enclosed drawings where:

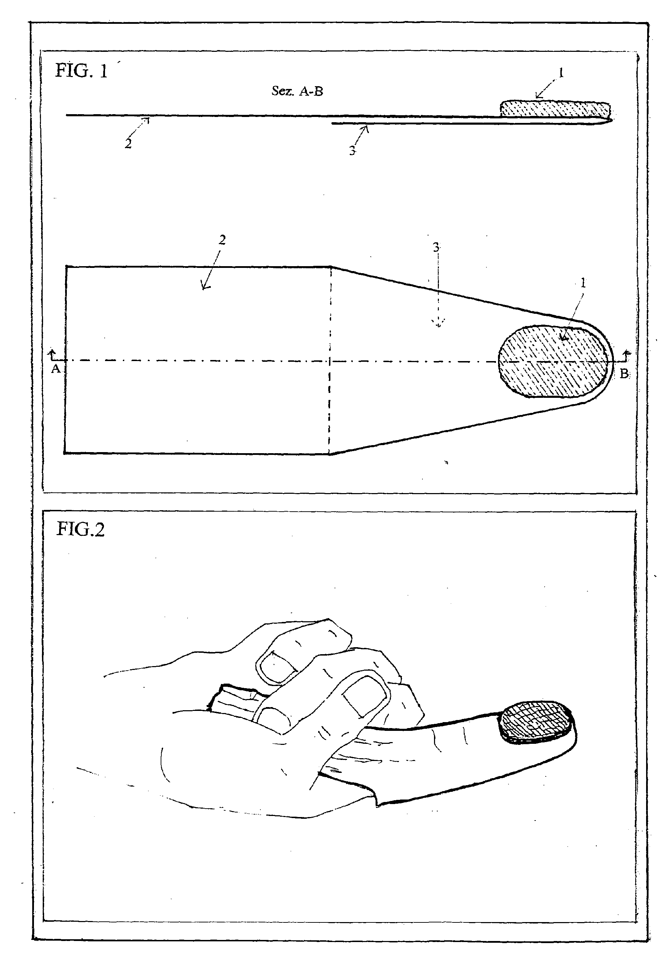

**fig. 1** and **main drawing** show the item in plan and in section, in its components, made by two layers of film and by the spongy material: (1) head of spongy material; (2) fore face of the item; (3) back face of the item. The front face, on the right of the drawing, sticking perimetrically to the back face, creates the frustum of cone where the finger can be put in; the remaining part forms the extension useful to be tightened in the hand so that the item will remain anchored to the finger while used; **fig. 2** shows, with a perspective drawing, how to wear the item.

The item is totally innovative both for ***functionality****,* for ***using form,*** for ***using occasion,*** beside the very small cost.
- **functionality:** it is easy to wear, anchoring itself to the forefinger and being blocked just clenching the fist; it contains inside itself the substance to apply, so to be totally self-sufficient; the changing density of the spongy material and its roughness, with an adequate consistency, guarantee an effectual action related to the use to be done.
- **using form:** its natural holding makes it very easy to use, without any limitations, and its subsequent adaptation to the movements of the forefinger makes it particularly efficacious in its action, also in relation to the extreme easiness to reach the more far and difficult points of the surface to treat.
- **using occasion:** the item doesn't have any limitation, as its very little volume, its easy transportability - even in the smallest pocket or inside the wallet in the space for credit cards- and its self-sufficiency make it useful in any occasion, everywhere, in any moment of the day, in any condition.

The item is highly reliable for hygiene, because it is disposable and in single or multiple sealed wrapping. It is made of natural or synthetic material, ( hygienically compatible with the several using forms), suitable for being manufactured as thin film; it has appropriate thickness, consistence and bigness, and it is made in shape of frustum of cone with rounded apex or in other shapes suitable to fit a finger. The item has also an extension useful to be tightened in the fist of the hand to make it firmly anchored to the finger while used. The substance to apply is put in the head itself of the item, on, inside or under it. The active area of the item could be more or less thick and with bigger or smaller roughness, given by proper processing of the spongy material, so to grant bigger efficaciousness and using care respecting the surface to be treated. Finally the item could be used - differentiating the contents of the reservoir with the inclusion of pastes, salves, unguents, gels, crèmes, powders (lyophilizated too), disinfectants, medicinal drugs, gelatines, liquids, chemical or natural compounds of different kind and any other substance useful for manual application to massage, to rub, to spread, to tampon, etc.- to increase the action of the above mentioned substances, because the massage facilitates their application emphasizing the absorption.

## Claims

1. mono-use universal applier for pastes, salves, unguents, gels, crèmes, powders, disinfectants, liquids and any other substance susceptible of manual application for massage, rubbing, spreading, tamponing and similar, with a very small size.

2. claim as preceding numbered claim, **characterized in that** the item can be manufactured in any kind of material, natural or synthetic, biodegradable or not, hydrosoluble or not, suitable to be produced and worked in the shape of thin film.

3. claim as preceding numbered claims, **characterized in that** the item, once put-on, can follow the movements and articulations of the forefinger or, when necessary, of the forefinger and middle finger.

4. claim as preceding numbered claims, **characterized in that** the item could be included in a flat container, very thin, suitable to contain one or more units of the item, which, even folded up, has a thickness close to one millimetre; the wrapping could be in laminar aluminium, plasticized paper or any other material useful for wrapping.

5. claim as preceding numbered claims, **characterized in that** the item can be manufactured, contained and sold in single or in multiple wrapping.

6. claim as preceding numbered claims, **characterized in that** in a multiple wrapping the extraction of one unit arranges the extraction of the following one and so on.

7. claim as preceding numbered claims, **characterized in that** the spongy material can be of changeable density and roughness, and it can occupy an area more or less large on the active surface of the item.

8. claim as preceding numbered claims, **characterized in that** the item can be manufactured in standard size or in different sizes.

9. claim as preceding numbered claims, **characterized in that** the system of anchoring the item to the forefinger could consist, instead in the above mentioned expansion, in an adhesive area inside the frustum of cone where the finger has to be put in, protected by a flap to be pulled away before using.

10. claim as preceding numbered claims, **characterized in that** the substance to be applied may be contained, together with the spongy material, in the same reservoir front/back, with interspace and micro-openings with predisposed breakage depending from the smaller resistance of the sealing **in that** areas, so that the head and the substance to apply could become communicating.

11. claim as preceding numbered claims, **characterized in that** the substance to be applied may be contained in an accessory reservoir, distinct and adjoining the one containing the spongy material, separated from this one by areas with fixed in advance openings due to smaller resistance of the soldering in those areas, so that, thank to the pressure exercised by the forefinger on the surface to be treated, the substance can flow into the spongy material and by this can be applied for the specific use.

12. claim as preceding numbered claims, **characterized in that** the substance contained in the accessory reservoir can flow directly outside, through areas of smaller resistance, without passing through the main reservoir containing the spongy material.

13. claim as preceding numbered claims, **characterized in that** the accessory reservoir may be located in different areas of the item, specifically in perimetricial area around the main reservoir.

14. claim as preceding numbered claims, **characterized in that** the reservoir containing the substance to be applied may be divided in two or more sectors, each of them distinctly communicating with the spongy material, so that bicomponent or multi-component agents may be used.

15. claim as preceding numbered claims, **characterized in that** each reservoir may contain different substances, so to consent the use of the item in different applications, for medical use, cosmetic, veterinary, hygienic or other suitable uses to receive advantage by the utilization of the item itself.

16. claim as preceding numbered claims, **characterized in that** the item may be manufactured and enclosed in sterilizable wrapping for those applications in sanitary field that could need sterilization.

17. claim as preceding numbered claims, **characterized in that** the wrapping of the item may contain accessories for the use the item is deputy, saving the characteristic of very small size, or the wrapping itself may be enclosed, together with other instruments and/or accessories of changeable greatness, in other bigger wrappings for complex uses.
